Europäisches Patentamt

European Patent Office

Office européen des brevets

(19)

(11) Numéro de publication: **0 114 560**
**B1**

(12) **FASCICULE DE BREVET EUROPEEN**

(45) Date de publication du fascicule du brevet: **02.09.87**

(51) Int. Cl.⁴: **A 61 F 13/08,** A 61 F 5/01

(21) Numéro de dépôt: **83420188.1**

(22) Date de dépôt: **15.12.83**

(54) **Chevillère de rééducation.**

(30) Priorité: **15.12.82 FR 8221375**
**07.07.83 FR 8311648**

(43) Date de publication de la demande:
**01.08.84 Bulletin 84/31**

(45) Mention de la délivrance du brevet:
**02.09.87 Bulletin 87/36**

(84) Etats contractants désignés:
**AT BE CH DE GB IT LI NL SE**

(56) Documents cités:
**DE-C- 696 697**
**FR-A-1 263 391**
**FR-A-2 364 647**
**FR-A-2 494 582**
**US-A-4 166 460**
**US-A-4 313 433**
**US-A-4 367 733**

(73) Titulaire: **Tricotage Elastique du Forez, S.A.**
**219 route d'Andrézieux**
**F-42170 Saint-Just-Saint-Rambert (FR)**

(72) Inventeur: **Bertheas, Michel**
**1, rue Jean Baptiste Marcet**
**F-42170 St-Just-St-Rambert (FR)**

(74) Mandataire: **Dupuis, François**
**Cabinet Charras 3 Place de l'Hôtel-de-Ville**
**F-42000 St.Etienne (FR)**

Courier Press, Leamington Spa, England.

## Description

La majorité des entorses de la cheville intéresse le faisceau ligamentaire externe, composé schématiquement de trois ligaments, antérieur, moyen et inférieur, qui s'attachent sur la malléole péronière d'une part, et sur l'astragale et le calcanéum d'autre part.

La fréquence des entorses externes de la cheville par rapport aux déchirures ligamentaires internes provient d'une plus grande mobilité du secteur articulaire externe, les contraintes exercées par les déplacements en terrain accidenté, les sauts, la course et les bondissements répétés, sollicitent énormément l'articulation tibiotarsienne. Le maintien de l'astragale dans la demi mortaise tibio-périonière est assuré de façon passive par les ligaments, et de façon active par les muscles péroniers à l'extérieur de l'articulation. Ces muscles réglent en particulier la coaptation du secteur articulaire externe de façon active et grâce au contrôle musculaire automatique. Lorsque pour des raisons diverses et, en particulier, de fatigue, ce contrôle musculaire automatique diminue, le pied risque davantage de se poser sur le sol en attitude vicieuse, et en raison de la laxité articulaire du segment externe, un baillement brutal de l'articulation vers l'extérieur au moment de la réception d'un saut, ou lors de la réception en appui unipodal en terrain accidenté, va se produire, ce baillement étant limité de façon prédominante seulement par le faisceau ligamentaire externe, les muscles n'assurant plus, en raison de la fatigue, la coaptation active de l'articulation. C'est ainsi qu'un varus brutal échappant au contrôle des muscles péroniers va provoquer une déchirure des ligaments, d'autant plus importante que le baillement sera important en amplitude.

Lorsqu'il y a lésion ligamentaire externe, le traumatisme local provoque une inhibition plus ou moins importante des muscles péroniers en particulier, et, outre la douleur qui interdit l'appui en charge, il existe une instabilité par perte de contrôle musculaire automatique qui peut rendre la marche impossible.

Selon la gravité des lésions ligamentaires, une immobilisation platrée est indiquée. Elle sera d'autant plus longue que la lésion est importante. En régle générale, l'immobilisation plâtrée peut aller de dix jours à 45 jours, en moyenne 21 à 25 jours.

Si l'immobilisation plâtrée est parfois la bonne solution pour cicatriser les lésions ligamentaires de la cheville, elle n'est pas dénuée d'inconvénients en raison de l'ankylose articulaire qu'elle présente et l'atrophie musculaire qu'elle provoque. Ainsi, plus l'immobilisation plâtrée est longue, plus la récupération sera tardive après cicatrisation, d'où la nécessité d'une kinésithérapie qui va permettre de retrouver la mobilité articulaire et la force musculaire ainsi que sa puissance, et sa faculté à exercer le contrôle musculaire automatique assurant la stabilité articulaire dans le mouvement.

Lorsque la lésion ligamentaire est bénigne, il n'y a pas besoin d'immobilisation plâtrée; cela ne veut pas dire pour autant que, dans les deux semaines qui suivront la lésion ligamentaire, la cheville sera fonctionnelle, en particulier pour la course, le saut, les sprints, c'est-à-dire les actes sportifs. La lésion, bien que bénigne, limitera, d'une part le mouvement en raison des douleurs et, surtout, présentera des risques d'instabilité en particulier par inhibition musculaire, ce qui provoquera des entorses à répétition risquant d'aggraver les lésions ligamentaire, donc de prolonger l'impotence fonctionnelle.

Le problème à résoudre est donc, pour les entorses plâtrées, de permettre la reprise fonctionnelle articulaire et musculaire, dans les délais les plus courts possibles, mais dans les meilleures conditions de confort et, surtout de sécurité.

En ce qui concerne les entorses bénignes, il faut assurer le maintien de la fonction articulaire et musculaire pour la poursuite de l'entrainement ou pour la compétition, en limitant au maximum les risques d'instabilité.

Traditionnellement, l'insuffisance temporaire de ces ligaments est compensée au moyen d'un bandage enveloppant la cheville, ou au moyen d'une chevillère élastique exerçant une force de contention autour de la cheville. De telles chevillères sont, en général, formées par une chaussette en tricot élastique comportant, de part et d'autre d'une partie centrale apte à envelopper le talon et le cou-de-pied, des parties tubulaires aptes à envelopper respectivement le bas de la jambe et une partie du pied.

Le brevet des Etats Unis d'Amérique 4 313 433 décrit une chevillère composée d'une enveloppe élastique horizontalement et non élastique verticalement, fermée par laçage sur le cou-de-pied et sur laquelle sont fixées des bandes non élastiques aptes à passer séparément sur la face postérieure de la cheville et sur le cou-de-pied où leur extrémité est fixée par coopération avec des moyens complémentaires d'accrochage.

La chevillère décrite dans le brevet des Etats Unis d'Amérique 4 166 460 est composée d'une chaussette tubulaire entourant le bas de la jambe, le talon et le pied à l'exception des doigts, chaussette qui, élastique radialement, est associée à des baleines verticales, et des lanières inextensibles attachées sous la face inférieure de la chaussette, s'enroulant sur le cou-de-pied, autour de la cheville et autour de la base de la jambe et s'accrochant par leur extrémité près du sommet de la chaussette.

On cite également en égard de la revendication 5 au titre de l'état de la technique en raison de sa date de publication, le Brevet US 4 367 733. La chevillère décrite dans ce Brevet prévoit une bande élastique associée à une gaine, ladite bande étant agencée avec des moyens d'accrochage. Cette bande est fixée à la gaine en correspondance sous la voute plantaire et nécessite plusieurs enroulements en double recouvrement du cou-de-pied, du talon de sorte que sa mise en place est complexe, et est d'une gêne certaine pour l'utilisateur.

Ces chevillères combinent les avantages du bandage traditionnel et de la chevillère élastique mais contiennent mal la malléole externe et ne contribuent pas à une remise en état rapide.

La présente invention a pour but de fournir une chevillère qui, non seulement, compense l'instabilité de la cheville dûe à une déchirure ligamentaire, mais aussi permet le rééducation précoce et la pratique plus rapide de la marche, de la course, du saut et, de manière générale, de toute activité physique et sportive se pratiquant debout.

A cet effet, dans cette chevillère du type composé d'une chaussette extensible sur laquelle est fixée au moins une bande extensible dont au moins l'extrémité est munie de moyens d'accrochage aptes à coopérer avec des moyens complémentaires de cette chevillère, chaque bande ou fragment de bande enveloppant la chevillère est disposée localement et en service au dessus de l'un des logements attachés à la malléole externe de manière à procurer, une force de contention correspondant en direction, à celle du ligament sous-jacent, et dont l'intensité est réglée pour être au moins égale à celle de l'effort fourni normalement par ce ligament.

Lorsque la chevillère est en service, les bandes élastiques définissent des forces de contention qui, correspondant en direction et intensité à celles procurées par les ligaments sains de la malléole externe, pallient au fonctionnement du ou des ligaments lésés et compensent immédiatement l'instabilité ligamentaire. En d'autres termes, le port de cette chevillère quelques temps après cicatrisation anatomique de la déchirure permet en apportant un complément physique aux ligaments, d'assurer la rééducation articulaire et musculaire et la pratique de l'activité à l'origine de la déchirure.

Dans une forme d'exécution, la chevillère de rééducation est du type réalisée en matériau élastique ayant une forme tubulaire composée d'une partie centrale apte à envelopper le talon et le cou-de-pied et, de part et d'autre de celle-ci, de parties tubulaires aptes à envelopper le bas de la jambe et une partie du pied, chevillère sur laquelle sont fixées les extrémités de bandes dont les autres extrémités sont munies de moyens d'accrochage aptes à coopérer avec des moyens complémentaires de cette chevillère, ladite chevillère étant caractérisée en ce qu'elle comporte trois bandes extensibles qui, aptes à former sur la malléole externe tout ou partie des trois branches d'un Y centré sur la malléole, correspondant sensiblement à la disposition des ligaments de celle-ci, sont fixées par l'une de leurs extrémités sur la surface externe de la partie de la chevillère, qui est destinée à recouvrir la malléole interne, et sont munies à leurs extrémités libres desdits moyens d'accrochage aptes à coopérer, après passage de ces bandes respectivement derrière la chevillère, sous le pied et sur le cou-de-pied, avec lesdits moyens complémentaires disposés sur la surface externe de la partie de la chevillère, qui est destinée à recouvrir la malléole externe, cette disposition des bandes amenant à procurer en service des forces de contention correspondant, en direction, à celles des ligaments sains sous-jacents, et en intensité, au moins l'effort fourni normalement par les ligaments correspondants.

Bien que donnant entièrement satisfaction dans la plupart des cas, l'usage montre que pour certaines entorses, la contention ligamentaire s'avère insuffisante. Pour remédier à cela et permettre un bon soutien du ligament lésé, l'invention fournit une autre forme d'exécution des moyens engendrant les forces de contention compensatrices.

La chevillère de rééducation est du type réalisé en matériau élastique ayant une forme tubulaire composée d'une partie centrale apte à envelopper le talon et le cou-de-pied et, de part et d'autre de celle-ci, de parties tubulaires aptes à envelopper le bas de la jambe et une partie du pied, chevillère sur laquelle est fixée l'extrémité d'une bande extensible dont au moins l'autre extrémité, sur sa face en regard de la chevillère, est munie de premiers moyens d'accrochage aptes à coopérer avec des moyens d'accrochage complémentaires de cette chevillère, disposés en certains points le long de la bande, caractérisée en ce que la bande est fixée par son extrémité sur la partie de la chevillère apte à venir immédiatement au-dessus, vu dans le sens longitudinal de la jambe, de la malléole externe et en ce que les moyens d'accrochage complémentaires sont formés de deuxièmes moyens d'accrochage disposés sur l'extrémité fixe de la bande et/ou de troisièmes moyens d'accrochage disposés entre ces deux extrémités sur la partie approximativement médiane de la longueur de la bande sur sa face externe opposée à la chevillère, ladite bande étant apte à former, en étant étirée, un huit passant sous la voûte plantaire, croisant sur le cou-de-pied et contournant la face postérieure de la cheville, au-dessus de l'interligne articulaire tibio-tarsien, en procurant, au niveau de la malléole et en raison de sa tension interne et de sa disposition, des forces de contention s'exerçant selon trois lignes de force formant un Y et correspondant en direction à celles des ligaments sous-jacents et dont l'intensité est réglée pour être au moins égale à celle de l'effort fourni normalement par les ligaments.

La bande élastique forme un huit autour de l'articulation tibio-tarsienne, et réalise ainsi une contention antéro-externe prédominante à l'endroit où se constituent le plus souvent les entorses ligamentaires. Ces doubles contentions élastiques, assurées par le chaussette en tricot et le bandage permettent les mouvements de flexion et d'extension de l'articulation sans supprimer totalement la faible amplitude en varus qui est nécessaire à l'adaptation du pied dans le mouvement. Plus précisémment, différentes parties de la bande forment sur la malléole externe, et comme montré dans la première forme d'exécution, des forces de contention correspondant en direction à celles des ligaments sous-jacents, et en intensité, au moins à l'effort fourni, par le ligament correspondant. Cette intensité est déterminée par la tension élastique communiquée à la bande, tension qui est déterminée de manière à ne pas provoquer une striction de la cheville en risquant de gêner la circulation veineuse.

D'autres caractéristiques et avantages ressortiront bien de la description qui suit en référence au dessin schématique représentant à titre d'exemples non limitatifs, plusieurs formes d'exécution de cette chevillère.

Les figures 1 et 2 sont des vues de côté en élévation montrant en position d'utilisation une première forme d'exécution de cette chevillère, respectivement de son côté externe et de son côté interne,

La figure 3 est une vue partielle de côté en élévation montrant la face externe de la chevillère des figures 1 et 2 et le moyen d'accrochage des brides.

La figure 4 est une vue en perspective montrant une autre forme d'exécution de cette chevillère lorsqu'elle est mise en place sur une petite cheville.

La figure 5 est une vue de côté en élévation montrant la chevillère à l'état non montée,

La figure 6 est une vue en coupe suivant 6—6 de la figure 5, montrant à échelle agrandie les troisièmes moyens d'accrochage,

La figure 7 est une vue de côté en perspective montrant la chevillère des figures 4 à 6 lorsqu'elle est montée sur une cheville forte,

La figure 8 est une vue partielle de côté en élévation montrant à échelle agrandie une variante de réalisation des troisièmes moyens d'accrochage.

De façon connue, la chevillère (2) réalisée en tricot ou tout autre textile élastique, comprend une partie centrale (2a) apte à envelopper le talon et le cou-de-pied, une partie tubulaire supérieure (2b) apte à envelopper le bas de la jambe, et une partie inférieure (2c) apte à envelopper une partie du pied.

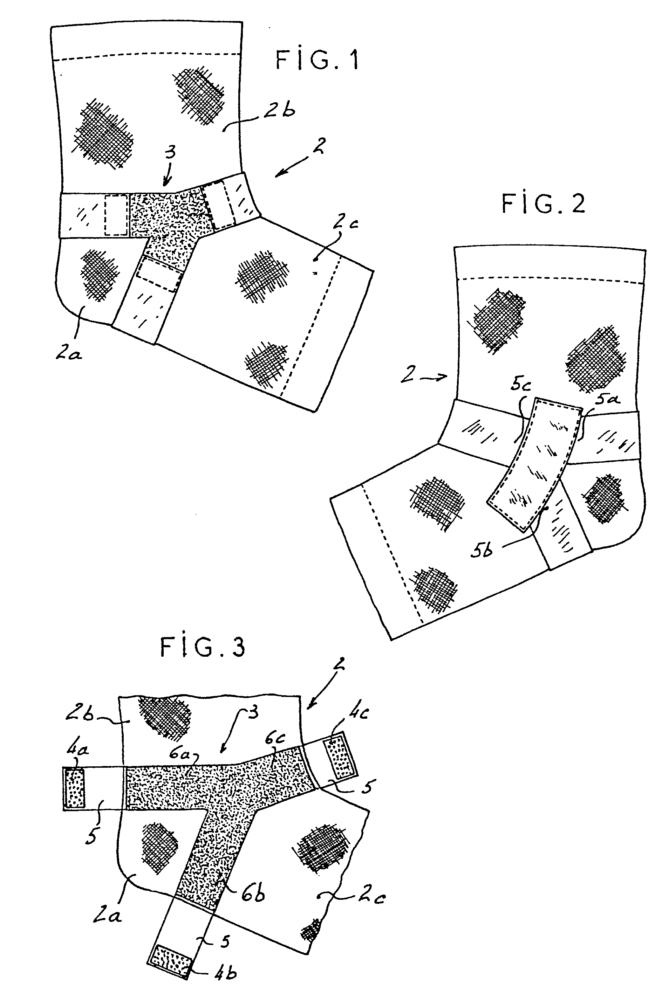

Dans la forme d'exécution représentée figures 1 à 3, la partie centrale (2a) de la chevillère et, plus particulièrement, la zone de celle-ci destinée à recouvrir la malléole externe de la cheville, est solidaire de moyens d'accrochage (3) aptes à coopérer avec des moyens d'accrochage complémentaires (4a—4b) et (4c) ménagés aux extrémités libres de trois bandes élastiques (5). Comme montré à la figure 2. Les bandes élastiques (5) sont fixées par leur autre extrémité, respectivement (5a—5b) et (5c) sur la partie de la chevillère apte à recouvrir la malléole interne de la cheville, c'est-à-dire sur la partie qui est opposée à celle munie des moyens d'accrochage (3).

Dans la forme d'exécution représentée, les moyens d'accrochage (3), solidaires de la chevillère (2), sont composés par trois bandes, respectivement (6a—6b) et (6c), d'une nappe de bouclettes apte à coopérer, chacune séparément, avec l'une des nappes de crochets constituant les moyens d'accrochage (4a—4b) et (4c) des brides élastiques (5). Les bandes (6a), (6b) et (6c) sont réparties de manière à former un Y correspondant à l'Y formé par les extrémités libres des trois bandes élastiques (5). Plus précisémment, les trois branches de cet Y ont des directions et dispositions qui correspondent à celles des trois ligaments de la malléole externe. Deux des branches (6a—6b) de cet Y divergent en direction du talon, tandis que la branche (6c) est orientée à l'opposé de ce talon.

Lorsque la chevillère est placée sur cette cheville blessée, le talon dont est munie sa partie centrale (2a) assure son positionnement parfait et garantit ainsi que la partie centrale des moyens d'accrochage (3) coïncide avec la partie centrale de la malléole externe. Après mise en place, et comme montré à la figure 1, les bandes élastiques (5) qui, enveloppant respectivement le cou-de-pied, la plante des pieds et la partie supérieure ou postérieure du talon, délimitent à leur tour une sorte de Y qui correspond sensiblement à celui formé par les trois ligaments associés à la malléole externe qu'il faut rééduquer.

Le recours aux bandes (5a—6b—6c) permet de constituer un moyen d'assemblage entre la chevillère et les extrémités des bandes élastiques (5) permettant de régler aisément la position d'accrochage de l'extrémité de chaque bande (5), et par conséquent, la tension communiquée à chacune de ces bandes.

Ainsi, lorsque la chevillère est placée sur une cheville affaiblie par un accident traumatique et, notamment, par une déchirure de l'un ou de plusieurs de ses ligaments externes, la tension communiquée à chaque bande (5) peut être parfaitement ajustée afin que la force de réaction communiquée par son élasticité soit au moins égale et, de préférence, supérieure à l'effort fourni normalement par le ligament, ce qui permet un réglage précis selon le siège de la lésion.

En pratique, l'utilisateur de la chevillère règle de lui-même la tension jusqu'à ce que celle-ci soit suffisante pour qu'il ne sente plus la faiblesse dûe à la déchirure ligamentaire.

Grâce à cet agencement permettant d'exercer sur le ou les ligaments déficients des efforts parfaitement orientés et réglés en intensité, la rééducation de la cheville s'effectue très rapidement ainsi que se récupère plus vite l'activité ou l'exercice qui est à l'origine de la déchirure et, à défaut la pratique des autres activités humaines s'exerçant debout.

Bien entendu, du fait de sa structure particulière et de son affectation à la rééducation tibiotarsienne, cette chevillère est composée de deux éléments, respectivement droit et gauche, pour coopérer avec le pied droit ou le pied gauche de l'individu.

Il est évident, que les moyens d'accrochage, constitués dans la forme d'exécution représentée par des nappes de bouclettes et de crochets, peuvent être remplacés par tout autre moyen équivalent.

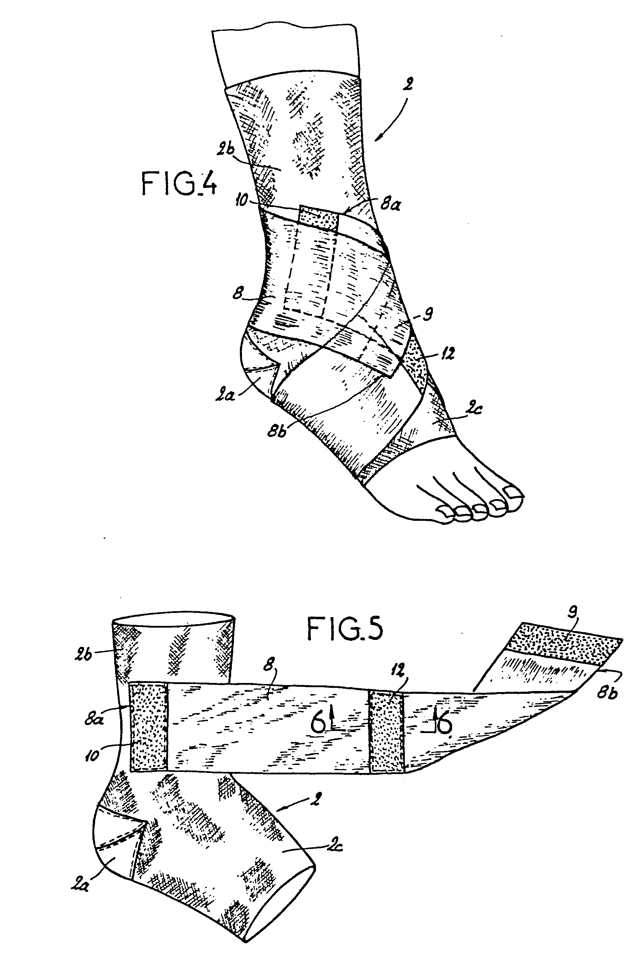

Dans la forme d'exécution représentée aux figures 4 à 8, la chaussette (2) est solidaire d'une seule bande élastique extensible longitudinalement (8) dont l'extrémité postérieure (8a) est fixée sur la chaussette dans la partie de celle-ci apte à venir au-dessus de la face postéro-externe de la malléole péronière et du quart distal du péroné. Cette bande (8) est munie à son extrémité libre (8b) de premiers moyens d'accrochage (9) aptes à coopérer, soit avec des deuxièmes moyens d'accrochage (10), fixés à l'extrémité postérieure de la bande, soit avec des troisièmes moyens d'accrochage (12). Ceux-ci sont fixés entre les deux extrémités de la bande et de manière à venir sur la partie antérieure du pied.

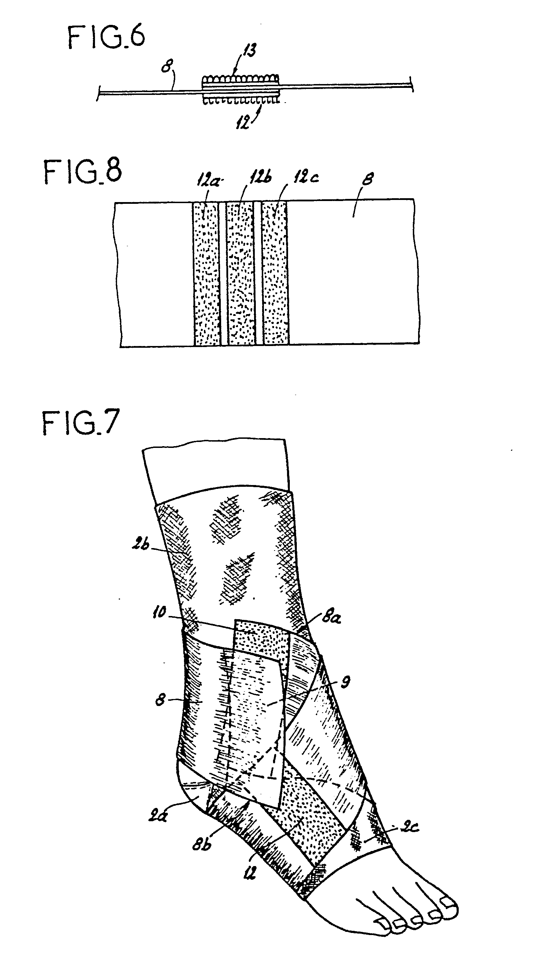

Les moyens d'accrochage sont constitués par des nappes de tissus à crochets ou à bouclettes aptes à coopérer ensemble. Lorsqu'elle est mise en place sur la cheville, la bande élastique (8) est étirée et est disposée de manière à passer sur le cou-de-pied, à descendre le long de la face interne du tarse, à passer en avant de la malléole tibiale interne, puis sous la voûte plantaire, au niveau du tarse, pour remonter par la face externe en avant de la malléole péronière. Elle passe ensuite à nouveau sur le cou-de-pied, en croisant sa propre trajectoire, passe au-dessus de la malléole tibiale interne, contourne la face postérieure de la cheville, au-dessus de l'interligne articulaire tibio-tarsien, pour venir s'accrocher solidement par engagement de ses moyens d'accrochage (9) dans ceux (10) fixés sur la face antéro-externe de la cheville ou, dans le cas de petites chevilles, sur les troisièmes moyens d'accrochage (12) disposés sensiblement sur le cou-de-pied. Dans le cas de chevilles fortes, et comme montré à la figure 7, les moyens d'accrochage (9) peuvent coopérer à la fois avec les deuxièmes moyens d'accrochage (10) et avec les troisièmes moyens d'accrochage (12).

Il apparait donc que, quelle que soit la dimension de la cheville, l'accrochage de la bande est toujours assuré parfaitement, et permet un réglage d'intensité de la tension communiquée à cette bande.

En raison de sa trajectoire en huit, cette bande exerce des forces de contention qui ont des directions sensiblement identiques à celles des ligaments sous-jacents. De plus, par son passage sur la face postérieure de la cheville, la bande assure également la contention du ligament postérieur.

Une telle chevillère permet de s'opposer aux baillements exagérés du compartiment externe de l'articulation. De préférence, et pour assurer un calage des deux boucles du huit formé par la bande, celle-ci comporte, sensiblement à hauteur des troisièmes moyens d'accrochage (12), mais sur la face opposée de la bande, des quatrièmes moyens d'accrochage (13) constitués, de préférence, par une nappe de tissus à crochets. Aussi, comme montré plus en détail à la figure 4, lorsque la bande forme un huit, les moyens d'accrochage (13) coopèrent avec la partie sous-jacente de la bande, au point de croisement des deux boucles, et assurent le calage du montage sur la cheville.

Enfin, comme montré à la figure 8, dans une variante de réalisation et de manière à faciliter les réglages d'accrochage, les troisièmes moyens d'accrochage (12) sont constitués par trois bandes transversales juxtaposées respectivement (12a), (12b) et (12c).

Il est évident que les bandes des moyens d'accrochage peuvent présenter toutes autres formes et dispositions que celles décrites ci-dessus uniquement à titre d'exemple.

La chevillère de rééducation constituée par la chaussette (2) et la bande élastique (8), constitue un système de double contention élastique permettant d'empêcher ou de diminuer l'oedème réactionnel aux entorses, mais aussi de suppléer aux carences ligamentaires.

Cette chevillère, destinée à permettre la reprise des fonctions naturelles de la cheville avec un meilleur confort, tout en diminuant considérablement le risque d'instabilité chez le sportif en particulier, peut être indiquée aussi dans les séquelles douloureuses d'entorses négligées, les oedèmes chroniques après lésion ligamentaire. Enfin, elle peut être portée à titre préventif chez des sujets particulièrement fragiles au niveau de l'articulation de la cheville.

**Revendications**

1. Chevillère (2) de rééducation du type réalisé en matériau élastique ayant une forme tubulaire composée d'une partie centrale (2a) apte à envelopper le talon et le cou-de-pied et, de part et d'autre de celle-ci, de parties tubulaires (2b, 2c) aptes à envelopper le bas de la jambe et une partie du pied, chevillère (2) sur laquelle sont fixées les extrémités (5a, 5b, 5c) de bandes (5) dont les autres extrémités sont munies de moyens d'accrochage (4a, 4b, 4c) aptes à coopérer avec des moyens complémentaires (3) de cette chevillère (2), caractérisée en ce que la chevillère (2) comporte trois bandes extensibles (5) qui, aptes à former sur la malléole externe tout ou partie des trois branches d'un Y centré sur la malléole, correspondant sensiblement à la disposition des ligaments de celle-ci, sont fixées par l'une de leurs extrémités (5a, 5b, 5c) sur la surface externe de la partie de la chevillère, qui est destinée à recouvrir la malléole interne, et sont munies à leurs extrémités libres desdits moyens d'accrochage (4a, 4b, 4c) aptes à coopérer, après passage de ces bandes respectivement derrière la chevillère, sous le pied et sur le cou-de-pied, avec lesdits moyens complémentaires (3) disposés sur la surface externe de la partie de la chevillère, qui est destinée à recouvrir la malléole externe, cette disposition des bandes (5) amenant à procurer en service des forces de contention correspondant, en direction, à celles des ligaments sains sous-jacents, et en intensité, au moins l'effort fourni normalement par les ligaments correspondants.

2. Chevillère selon la revendication 1, caractérisée en ce que les moyens d'accrochage (4a, 4b, 4c) de chaque bande (5) sont indépendants de ceux des autres bandes.

3. Chevillère selon l'une quelconque des revendications 1 ou 2, caractérisée en ce que les moyens d'accrochage (3, 4a, 4b, 4c) sont constitués par des nappes de tissus à crochets et/ou à bouclettes aptes à coopérer ensemble.

4. Chevillère selon la revendication 3, caractérisée en ce que la nappe de tissus à bouclettes fixée sur la chevillère a une forme en "Y" dont deux des branches (6a, 6b) divergent en direction du talon (2a), tandis que l'autre branche (6c) est orientée à l'opposé du talon (2a).

5. Chevillère de rééducation du type réalisé en matériau élastique ayant une forme tubulaire composée d'une partie centrale (2a) apte à envelopper le talon et le cou-de-pied et, de part et d'autre de celle-ci, de parties tubulaires (2b, 2c) aptes à envelopper le bas de la jambe et une partie du pied, chevillère sur laquelle est fixée l'extrémité (8a) d'une bande extensible (8) dont au moins l'autre extrémité (8b), sur sa face en regard de la chevillère, est munie de premiers moyens d'accrochage (9) aptes à coopérer avec des moyens d'accrochage complémentaires de cette chevillère, disposés en certains points le long de la bande, caractérisée en ce que la bande (8) est fixée par son extrémité (8a) sur la partie (2b) de la chevillère (2) apte à venir immédiatement au-dessus, vu dans le sens longitudinal de la jambe, de la malléole externe et en ce que les moyens d'accrochage complémentaires sont formés de deuxièmes moyens (10) d'accrochage disposés sur l'extrémité fixe (8a) de la bande et/ou de troisièmes moyens d'accrochage (12) disposés entre ces deux extrémités sur la partie approximativement médiane de la longueur de la bande sur sa face externe opposée à la chevillère, ladite bande étant apte à former, en étant étirée, un huit passant sous la voûte plantaire, croisant sur le cou-de-pied et contournant la face postérieure de la cheville, au-dessus de l'interligne articulaire tibio-tarsien, en procurant, au niveau de la malléole et en raison de sa tension interne et de sa disposition, des forces de contention s'exerçant selon trois lignes de force formant un Y et correspondant en direction à celles des ligaments sous-jacents et dont l'intensité est réglée pour être au moins égale à celle de l'effort fourni normalement par les ligaments.

**Patentansprüche**

1. Fussknöchelbandage (2) für Heilgymnastickzwecke, die aus einem federnden Werkstoff rohrförmiger Gestaltung ausgeführt wird und aus den folgenden Bestandteilen besteht: einem Mittelteil (2a) zum Umwickeln um die Ferse und den Fusspann; und rohrförmigen Teilen (2b, 2c) beiderseitig dieses Mittelteils, zum Umwickeln um den Beinunterteil und einen Teil des Fusses, mit den auf dieser Fussknöchelbandage befestigten Enden (5a, 5b, 5c) von Bändern (5), derer andere Enden mit Hakenmitteln (4a, 4b, 4c) zum Mitwirken mit den Zusatzmitteln (3) dieser Fussknöchelbandage (2) versehen sind, dadurch gekennzeichnet, dass die Fussknöchelbandage (2) drei dehnbare Bänder (5) aufweist, die auf der Aussenmalleole die Gesamtheit oder einen Teil der drei Äste eines auf der Malleole zentrierten Y bilden können, die etwa der Anordnung der Muskelbänder der Malleole entsprechen, wobei diese dehnbaren Bänder (5) durch das eine ihrer Enden (5a, 5b, 5c) auf der Aussenoberfläche des zum Überdecken der Innenmalleole vorgesehenen Teils der Fussknöchelbandage befestigt werden, und an ihren freien Enden mit den besagten Hakenmitteln (4a, 4b, 4c) versehen sind, die nach Anlegen dieser Bänder beziehungsweise hinter die Fussknöchelbandage, unter den Fuss und über den Fusspann mit den besagten, auf der Aussenoberfläche eines Teils der Fussknöchelbandage angeordneten Zusatzmittel (3) mitwirken können, wobei der genannte Teil zum Überdecken der Aussenmalleole vorgesehen ist, und diese Anordnung der Bänder (5) während der Benützung des Fussknöchelbandage Zurückhaltenkräfte ausübt, die richtungsmässig den Zurückhaltenkräften der darunterliegenden Muskelbänder und kraftmässig am wenigsten der durch die entsprechenden Muskelbänder normalerweise entwickelten Anstrengung entsprechen.

2. Fussknöchelbandage nach Anspruch 1, dadurch gekennzeichnet, dass die Hakenmittel (4a, 4b, 4c) jedes einen Bands (5) unabhängig von den Hakenmitteln der anderen Bänder sind.

3. Fussknöchelbandage nach einem der Ansprüche 1 oder 2, dadurch gekennzeichnet, dass die Hakenmittel (3, 4a, 4b, 4c) aus behakten und/oder lockigen Gewebeschleiern bestehen, die zusammen mitwirken können.

4. Fussknöchelbandage nach Anspruch 3, dadurch gekennzeichnet, dass der auf der Fussknöchelbandage befestigte, aus Büscheln bestehende Gewebeschleier eine "Y"-Formgebung aufweist, wobei die zweie eine Äste (6a, 6b) dieses Y in der Richtung nach der Ferse (2a) auseinanderlaufen, während der andere Ast (6c) entgegengesetzt der Ferse (2a) orientiert ist.

5. Fussknöchelbandage für Heilgymnastikzwecke, die aus einem federnden Werkstoff rohrförmiger Gestaltung ausgeführt wird und aus den folgenden Bestandteilen besteht: einem Mittelteil (2a) zum Umwickeln um die Ferse und den Fusspann; und rohrförmigen Teilen (2b, 2c) beiderseitig dieses Mittelteils, zum Umwickeln um den Bandunterteil und einen Teil des Fusses, mit dem auf dieser Fussknöchelbandage befestigten Ende (8a) eines dehnbaren Bands (8) dessen anderes Ende (8b) am wenigstens, auf seiner Fläche entgegengesetzt der Fussknöchelbandage, mit den ersten Hakenmitteln (9) versehen ist, die mit den auf gewissen Stellen an diesem Band entlang angebrachten Zusatzmitteln dieser Fussknöchelbandage mitwirken können, dadurch gekennzeichnet, dass das Band (8) durch dessen Ende (8a) auf dem Teil (2b) der Fussknöchelbandage (2) befestigt ist, der unmittelbar oberhalb, in der Beinrichtung der Länge nach gesehen, der Aussenmalleole kommen kann; und dass die zusätzlichen Hakenmittel aus zweiten, auf dem freien Ende (8a) des Bands angebrachten Hakenmitteln (10) und/oder dritten Hakenmitteln (12) gebildet sind, die zwischen diesen zweien Enden auf dem nahezu mittigen Teil der Bandlänge auf der der Fussknöchelbandage entgegengesetzten Aussenoberfläche des Bands angeordnet sind, wobei das genannte Band bei dem Ausstrecken eine Acht zum Anlegen unter dem Fussbogen, zur Kreuzung auf dem Fusspann und zum Umlegen um die Hinterfläche des Fussknöchels oberhalb des artikularen Schienbeinwurzelabstands bilden kann, damit an der Höhe der Malleole und durch die Innenspannung und die Anordnung derselben Zurückhaltenkräfte erzeugt werden, die sich nach drei Kraftrichtungen ausüben, die eine Y bilden, und richtungsmässig der Richtung der darunterliegenden Muskelbänder entsprechen, wobei diese Zurückhaltenkräfte eine Stärke entwickeln, die so bemessen ist, dass sie am wenigstens der von den Muskelbändern normalerweise erzeugten Anstrengung gleichkommt.

**Claims**

1. Re-education ankle-pad (2) in the type made of elastic matérial having a tubular shape, consisting of a central portion (2a) capable of being wrapped up round the heel and the instep and at either side of this central portion, of tubular parts (2b, 2c) capable of being wrapped up round the lower part of the leg and a portion of the foot, there being fastened on said ankle-pad (2) the ends (5a, 5b, 5c) of bands (5) the other ends of which are provided with hooking means (4a, 4b, 4c) capable of co-operating with complementary means (3) of ankle-pad (2), which ankle-pad is characterized in that it has three extensible bands (5) capable of forming on the external malleolus the whole or a part of the three legs of an Y centered on the malleolus, corresponding approximately to the arrangement of the ligaments of same, said extensible bands being secured by one of the ends thereof (5a, 5b, 5c) to the external surface of the portion of the ankle-pad which is intended to cover the internal malleolus, and being provided at their free ends with the said hooking means (4a, 4b, 4c), capable of co-operating, after said bands have been passed respectively behind the ankle-pad, beneath the instep and on the instep, with said complementary means (3), arranged on the external surface of the portion of the ankle-pad which is intended to cover the external malleolus, said arrangement of the bands (5) permitting to originate retention forces during the use of the ankle-pad, said retention forces corresponding in direction with the direction of the sub-jacent healthy ligaments, and in intensity at least with the normal stress provided by the corresponding ligaments.

2. Ankle-pad as claimed in Claim 1, characterized in that the hooking means (4a, 4b, 4c) of each band (5) are independent from the hooking means of the other bands.

3. Ankle-pad as claimed in any one of Claim 1 or 2, characterized in that the hooking means (3, 4a, 4b, 4c) consist of sheets of hooked and/or looped fabrics capable of co-operating together.

4. Ankle-pad as claimed in Claim 3, characterized in that the sheet of fabrics in small loops secured to the ankle-pad is formed in the shape of an "Y" two legs (6a, 6b) of which spread out in the direction of the heel (2a), while the other leg (6c) is oriented opposite the heel (2a).

5. Re-education ankle-pad in the type made of elastic material having a tubular shape, consisting of a central portion (2a) capable of being wrapped up round the heel and the instep and at either side of this central portion of tubular parts (2b, 2c) capable of being wrapped up round the lower part of the leg and a portion of the foot, there being fastened on said ankle-pad the end (8a) of an extensible band (8), the other end (8b) at least of said band, on the face thereof opposite the ankle-pad, being provided with first hooking means (9) capable of co-operating with complementary hooking means of this ankle-pad, disposed at certain points along the band, said ankle-pad being characterized in that the band (8) is fastened at the end (8a) thereof on the portion (2b) of the ankle-pad (2) which is conveniently arranged to be placed immediately above, as seen in the longitudinal direction of the leg of the patient, the external malleolus, the complementary hooking means being made of second hooking means (10) disposed on the fixed end (8a) of the band and/or of third hooking means (12) disposed between the two ends of the band on the approximately medial portion of the length of the band on the external face thereof opposite the ankle-pad, said band being designed to form while being stretched an eight-shaped portion passing beneath the plantar arch, crossing on the instep and winding round the posterior face of the ankle, above the tibio-tarsal articular interspace, while providing at the level of the malleolus and by reason of the internal stretching and of the arrangement of the band various retention forces exerted along three lines of force which form an Y and correspond in direction to the directions of the sub-jacent ligaments, the intensity of said forces being so adjusted that they will be at least equal in intensity to the intensity of the normal stress provided by the ligaments.

FIG.1

2b

3

2

2c

2a

FIG.2

2

5c

5a

5b

FIG.3

2b

4a

4c

3

6c

6a

5

5

6b

2c

2a

5

4b

0 114 560

FIG.4

FIG.5

2

FIG.6

FIG.8

FIG.7